# EUROPEAN PATENT SPECIFICATION

(11) **EP 3 052 089 B1**
(45) Date of publication and mention of the grant of the patent: **30.08.2017**
(21) Application number: 14849334.9
(22) Date of filing: 01.09.2014
(51) Int. Cl.: A61K 9/20, A61K 31/167, A61K 31/135, A61P 25/04, A61P 29/00

(54) **TRAMADOL HYDROCHLORIDE AND PARACETAMOL ORALLY DISINTEGRATING COMPOSITION AND PROCESS FOR PREPARING THE SAME**
ZUSAMMENSETZUNG ZUR ORALEN DISINTEGRATION VON TRAMADOLHYDROCHLORID UND PARACETAMOL UND VERFAHREN ZUR HERSTELLUNG DAVON
COMPOSITION ORODISPERSIBLE CONTENANT DU CHLORHYDRATE DE TRAMADOL ET DU PARACÉTAMOL, ET PROCÉDÉ DE PRÉPARATION DE CETTE COMPOSITION

(30) Priority: 30.09.2013 IN 3098MU2013
(43) Date of publication of application: 10.08.2016
(73) Proprietor: Athena Drug Delivery Solutions Pvt Ltd., Andheri (East) Mumbai 400059 (IN)
(72) Inventor: B. CHAUDHARI, Mahendra, Maharashtra 400602 (IN); S. WADHWANI, Jagdish, Maharashtra 421005 (IN)
(74) Representative: Herrero & Asociados, S.L.
(86) International application number: PCT/IN2014/000568
(87) International publication number: WO 2015/044952

(56) References cited:
- WO-A1-2004/026308
- WO-A1-2010/062524
- CN-A- 1 732 910
- CN-A- 1 762 340
- CN-A- 1 762 340
- CN-A- 1 803 128
- CN-A- 1 803 128
- US-A1- 2004 247 677
- US-A1- 2004 247 677

## Description

### FIELD OF THE INVENTION

The present invention relates to tramadol hydrochloride and paracetamol orally disintegrating compositions; and process for preparing the same.

### BACKGROUND OF THE INVENTION

Tramadol hydrochloride is a synthetic opioid analgesic used to treat moderate to moderately severe pain; and paracetamol is a mild analgesic and is commonly used for the relief of headaches and other minor aches and pains and is a major ingredient in numerous cold and flu remedies. Conventional tramadol hydrochloride and paracetamol tablets have been available for a long time; however the disintegration of conventional tablets is much slower resulting in decreased efficacy of the medicine. Further, due to the bitter taste of the conventional tablets; elderly or children or people with an impaired swallowing function or people with decreased saliva production, the oral intake of conventional tablets are a cumbersome process.

Now, orally disintegrating tablets (ODTs) are available. ODTs differ from conventional tablets in that they are designed to be dissolved/disintegrated in an oral cavity rather than swallowed whole. Accordingly, ODTs have been developed such that the same medicine can be orally administered in a simple and effective manner to children, adults and people with impaired swallowing function and similar conditions. Even a patient who is not suffering from any swallowing limitation will find it much easier to take an ODT as compared to a conventional tablet. An additional reason to use an orally disintegrating tablet is the convenience of a tablet that can be taken without water.

In view of ease of administration, the ODTs have a rapid disintegration rate as compared to conventional tablets. However, rapid disintegration properties and high tablet hardness are generally contradicting properties, and therefore ODTs cause chips and cracks of the tablets when divided because of insufficient tablet hardness and high friability. Due to high disintegration and low hardness, even the cracking or disintegration problems are faced in the course of production and/or distribution.

Also, there are a number of production problems encountered when preparing tramadol hydrochloride and paracetamol compositions. One problem is that there are no efficient methods available for single process granulation of both the drugs. Another problem is that there are no efficient methods available for single taste mask coating for both the drugs. Due to these and other problems the process for manufacturing a combination composition is quite complex and unreliable.

For Example the patent application CN1083128 discloses orally disintegrating tablets comprising taste mask coated particles containing (a) taste mask coated granules of paracetamol and (b) taste mask coated particles of tramadol HCl.

Accordingly, there is a need for a tramadol hydrochloride and paracetamol composition that is simple, reliable and safe to manufacture, distribute and administer. Also, what is required is a tramadol hydrochloride and paracetamol composition that has excellent disintegration and dissolution properties while having an appropriate hardness to avoid any damage in the course of production and/or distribution. Further, what is required is a process for manufacturing a tramadol hydrochloride and paracetamol composition that provides for simple, reliable and efficient preparation of such composition.

### SUMMARY OF THE INVENTION

To achieve the foregoing and other objects and needs, the present invention provides a tramadol hydrochloride and paracetamol composition that provides better uniformity in low dosage and consistent reliability and efficacy of the tramadol hydrochloride and paracetamol. Also, the present invention provides a tramadol hydrochloride and paracetamol orally disintegration composition that has single process for granulation for both the drugs and further has a single taste mask coating for both the drugs.

In one aspect, the present invention provides tramadol hydrochloride and paracetamol orally disintegrating composition, comprising: about 2.5 to about 5 percent by weight of tramadol hydrochloride; about 25 to about 30 percent by weight of paracetamol; about 1.25 to about 1.75 percent by weight of colloidal silicon dioxide;about 0.25 to about 0.35 percent by weight of povidone K-30; about 4.5 to about 5.5 percent by weight of ethyl cellulose N7; about 1.5 to about 2 percent by weight of mannitol 25; about 20 to about 25 percent by weight of mannitol 400; about 20 to about 25 percent by weight of mannitol 35; about 9 to about 11 percent by weight of a disintegrant; about 3 to about 5 percent by weight of a sweetener; about 1 to about 1.5 percent by weight of a flavorant; and about 0.5 to about 1.5 percent by weight of a lubricant.

In another aspect, the present invention provides a process for preparation of a tramadol hydrochloride and paracetamol orally disintegrating composition. The process comprises: mixing tramadol hydrochloride, paracetamol and colloidal silicon dioxide to form a drug blend; mixing the drug blend and the alcoholic solution of povidone K-30 to form a granulation solution; drying, sizing and sieving of the granulation solution to form drug granules ;blending the drug granules with colloidal silicon dioxide to form lubricated drug granules; spraying a taste mask coating solution comprising ethyl cellulose N7 over lubricated drug granules to form coated drug granules; blending the coated drug granules with mannitol 25, mannitol 400, mannitol 35, a disintegrant, a sweetener, a flavorant, colloidal silicon dioxide and a lubricant to form a pre- compression composition; and compressing the pre-compression composition.

### BRIEF DESCRIPTION OF THE DRAWINGS

The advantages and features of the present invention will become better understood with reference to the following detailed description and claims taken in conjunction with the accompanying drawing, in which:
FIG. 1 illustrates a process flow for preparation of a tramadol hydrochloride and paracetamol orally disintegrating composition, in accordance with an exemplary embodiment of the present invention; and
FIG. 2 illustrates details of the granulation of process of FIG. 1;
FIG. 3 illustrates details of taste mask coating of FIG. 1;
FIG. 4 illustrates details of preparation of pre-compression composition of process of FIG. 1; and
FIG. 5 illustrates details of compression and packing steps of process of FIG. 1.

### DETAILED DESCRIPTION OF THE INVENTION

The use of terms "including," "comprising:," or "having" and variations thereof herein is meant to encompass the items listed thereafter and equivalents thereof as well as additional items. Further, the terms, "a" and "an" herein do not denote a limitation of quantity, but rather denote the presence of at least one of the referenced item.

The present invention provides a tramadol hydrochloride and paracetamol orally disintegrating composition (hereinafter referred to as the "composition"). The composition of the present invention has excellent efficacy, uniformity and reliability. Also, the composition has excellent disintegration and dissolution properties while having an appropriate hardness to avoid any disintegration in the course of production and/or distribution. In specific embodiments, as would be described below in the examples, the composition has a disintegrating time of about 3 minutes, while having hardness of about 40 Newton (N) to about 50 Newton (N).

The composition of the present invention comprises tramadol hydrochloride, paracetamol, colloidal silicon dioxide (aerosil 200), povidone K-30 (PVP K-30), ethyl cellulose N7, mannitol 25 (pearlitol 25), mannitol 400 (pearlitol 400), mannitol 35 (pearlitol 35), a disintegrant, a sweetener, a flavorant, and a lubricant. In one embodiment the composition comprises: about 2.5 to about 5 percent by weight of a tramadol hydrochloride; about 25 to about 30 percent by weight of paracetamol; about 1.25 to about 1.75 percent by weight of colloidal silicon dioxide; about 0.25 to about 0.35 percent by weight of povidone K-30; about 4.5 to about 5.5 percent by weight of ethyl cellulose N7; about 1.5 to about 2 percent by weight of mannitol 25; about 20 to about 25 percent by weight of mannitol 400; about 20 to about 25 percent by weight of mannitol 35; about 9 to about 11 percent by weight of a disintegrant; about 3 to about 5 percent by weight of a sweetener; about 1 to about 1.5 percent by weight of a flavorant; and about 0.5 to about 1.5 percent by weight of a lubricant.

As used herein, paracetamol (chemically named N-acetyl-p-aminopheno) refers to the widely used over-the-counter analgesic (pain reliever) and antipyretic (fever reducer). As used in the present invention, paracetamol is obtained from Sri Krishna Pharmaceuticals. Paracetamol is a mild analgesic and is commonly used for the relief of headaches and other minor aches and pains and is a major ingredient in numerous cold and flu remedies. In combination with opioid analgesics, paracetamol can also be used in the management of more severe pain such as post-surgical pain and providing palliative care in advanced cancer patients.

As used herein, tramadol hydrochloride (chemically named as (±)cis-2-[(dimethylamino)methyl]-1-(3methoxyphenyl) cyclohexanol hydrochloride) refers to a white, bitter, crystalline and odorless powder which is readily soluble in water and ethanol. It centrally acts as a synthetic opioid analgesic used to treat moderate to moderately severe pain. The drug has a wide range of applications, including treatment of rheumatoid arthritis, restless legs syndrome, motor neurone disease and fibromyalgia. As used in the present invention, tramadol hydrochloride is obtained from Jubilant Organosys.

As used herein, colloidal silicon dioxide refers to a fumed silica prepared by vapour-phase hydrolysis of a silicon compound, such as silicon tetrachloride. The product itself is usually a submicron, fluffy, light, loose, bluish-white, odourless and tasteless amorphous powder which is commercially available from a number of sources, including Cabot Corporation (under the trade name Cab-O-Sil®); Degussa, Inc. (under the trade name Aerosil®); Huber Engineered Materials (Huber GL100 and GL200®); Wacker (Wacker HDK ®); and E.I. DuPont & Co. As used in the present invention, colloidal silicon dioxide is obtained from Evonik & Roquette. Colloidal silicon dioxide is also known as Aerosil, colloidal silica, fumed silica, light anhydrous silicic acid, silicic anhydride, and silicon dioxide fumed, among others. In one embodiment, the colloidal silicon dioxide used is Aerosil 200. A variety of commercial grades of colloidal silicon dioxide are produced by varying the manufacturing. Also, the colloidal silicon dioxide is used herein as a glidant that serves as a material for improving powder flow since colloidal silicon dioxide is inert and doesn't dissolve in water. Also, the colloidal silicon dioxide is used herein as a lubricant.

Povidone K-30 is also commonly known as (PVP K-30). Povidone (polyvinylpyrrolidone, PVP) is used in the pharmaceutical industry as a synthetic polymer vehicle for dispersing and suspending drugs. It has multiple uses, including as a binder for tablets and capsules, a film former for ophthalmic solutions, to aid in flavoring liquids and chewable tablets, and as an adhesive for transdermal systems. The PVP K-30 has the molecular formula of (C₆H₉NO)ₙ and appears as a white to slightly off-white powder. PVP K-30 formulations are widely used in the pharmaceutical industry due to their ability to dissolve in both water and oil solvents. As used in the present invention, PVP K-30 is obtained from BASF.

As used herein, ethyl cellulose N7 is the non-iconic ethyl ether of cellulose, soluble in wide range of organic solvents. The ethyl cellulose N7 is used as a non-swellable, insoluble component in matrix or coating systems. When water-soluble binders cannot be used in dosage processing because of water sensitivity of active ingredient, the ethyl cellulose is often chosen. The ethyl cellulose N7 can be used to coat one or more active ingredient of a tablet to prevent them from reacting with other materials or with one another. It can prevent discoloration of easily oxidizable substances such as ascorbic acid, allowing granulations for easily compressed tablets and other dosage forms. It can be used on its own or in combinations with water-soluble polymers to prepare sustained release film coating that are frequently used for the coating of micro-particles, pellets and tablets. As used in the present invention, ethyl cellulose N7 is obtained from Colorcon and Aqualon.

As used herein, mannitol 25 refers to a white, crystalline sugar alcohol with the chemical formula (C₆H₈(OH)₆). As used in the present invention, mannitol 25 is in the form of "mannitol fine" (also known as Pearlitol 25) is obtained from Roquette. The Pearlitol range offers a unique blend of exceptional physical and chemical stability, with great organoleptic, non-carcinogenic, sugar-free properties. Together with its versatile powder properties, it is the key to a wide range of oral and injectable applications, and for use in different processes (wet or dry granulation, direct compression, compaction or freeze-drying).

As used in the present invention, mannitol 400 (also known as Pearlitol 400) is obtained from Roquette. Mannitol 400 used herein is in the form of "mannitol granular". The Pearlitol range offers a unique blend of exceptional physical and chemical stability, with great organoleptic, non-carcinogenic, sugar-free properties. Together with its versatile powder properties, it is the key to a wide range of oral and injectable applications, and for use in different processes (wet or dry granulation, direct compression, compaction or freeze-drying).

As used in the present invention, mannitol 35 (also known as Pearlitol 35) is obtained from Roquette. Mannitol 35 used herein is in the form of "mannitol powder". The Pearlitol range offers a unique blend of exceptional physical and chemical stability, with great organoleptic, non-carcinogenic, sugar-free properties. Together with its versatile powder properties, it is the key to a wide range of oral and injectable applications, and for use in different processes (wet or dry granulation, direct compression, compaction or freeze-drying).

In one embodiment, the disintegrant is polyplasdone XL. As used herein polyplasdone XL belongs to the category of polyplasdone crospovidones that are used in drug formulations. Not only are polyplasdone crospovidones highly effective tablet disintegrants; Polyplasdone superdisintegrants can improve the rate and extent of drug dissolution. As used herein, polyplasdone XL aids in increasing the dissolution rate of poorly soluble drugs compared with other superdisintegrants. As used in the present invention, the polyplasdone XL is obtained from BASF.

As used herein, the sweeteners can include aspartame, saccharin sodium, dipotassium glycyrrhizinate, stevia, thaumatin, acesulfame K, sucralose, and combinations of the foregoing. In one embodiment, the sweetener is aspartame. Aspartame is an artificial, non-saccharine sweetener used as a sugar substitute in some foods and beverages. Aspartame is a methyl ester of the aspartic acid/phenylalanine dipeptide. As used in the present invention, the aspartame is obtained from Nutra sweets.

The flavoring agents that can be used include natural and artificial flavors. These flavors may be one or more of synthetic flavor oils and flavoring aromatics, and/or oils, oleo resins and extracts derived from plants, leaves, flowers, fruits, etc., and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. Also useful are artificial, natural or synthetic fruit flavors such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth. These flavorings can be used individually or in admixture. Commonly used flavors also include mints such as peppermint, artificial vanilla, cinnamon derivatives, and various fruit flavors, whether employed individually or in admixture. Flavorings such as aldehydes and esters including cinnamyl acetate, cinnamaldehyde, citral, diethylacetal, dihydrocarvyl acetate, eugenyl formate, p-methylanisole, and so forth may also be used. In one embodiment, the flavorant is mint rootbeer. As used in the present invention, the mint rootbeer is obtained from Pharmarome.

Lubricants can be added to granules both during the final mixing phase before compression and during granulation. Among the traditional solid lubricants, calcium, magnesium, and zinc salts of stearic acid, partially hydrogenated vegetable oils, polyethylene glycols, polyoxyethylene monostearate, talc, sodium benzoate, sodium laurylsulfate, magnesium oxide can be used. In one embodiment lubricant is used in the blending applications for anti-adherent activity (i.e., prevent sticking to punch faces and die walls), glidant activity (i.e., improve the flowability of the powder or granules), and lubricant activity (i.e., reduce friction, transfer heat, and prevent corrosion during the process). In one embodiment, the lubricant is magnesium stearate.

As used herein, magnesium stearate refers to a white powdered substance having the chemical formula Mg(C₁₈H₃₅O₂)₂. It is a salt containing two equivalents of stearate (the anion of stearic acid) and one magnesium cation (Mg²⁺). Magnesium stearate is used herein to serve as a lubricating medium for the composition. Magnesium stearate prevents ingredients from sticking to manufacturing equipment during the compression of chemical powders into solid tablets. Magnesium stearate has advantages over other lubricants because of its high melting temperature, high lubricity at a low concentration, large covering potential, general acceptance as safe, nontoxicity, and its excellent stability profile. As used in the present invention, the magnesium stearate is obtained from Ferro (Portugal).

Further, the present invention provides a process for preparation of the tramadol hydrochloride and paracetamol orally disintegrating composition (hereinafter referred to as process). The process employs a single process for granulation of both the drugs; and a process single taste mask coating for both the drugs.

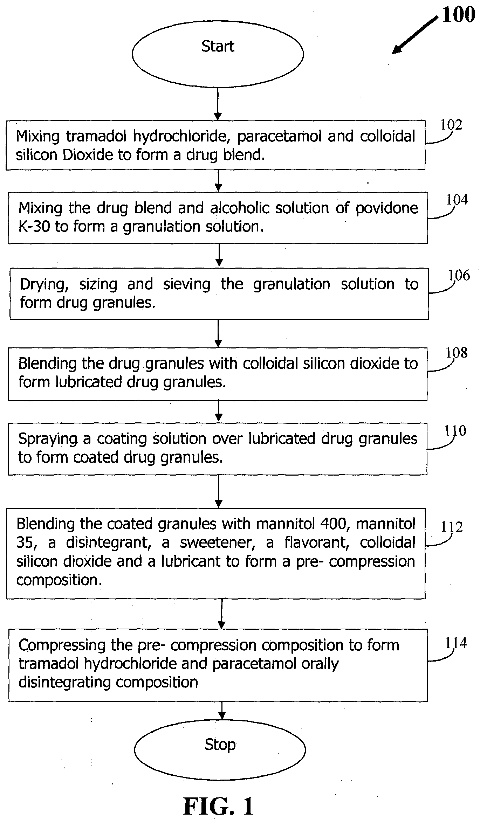

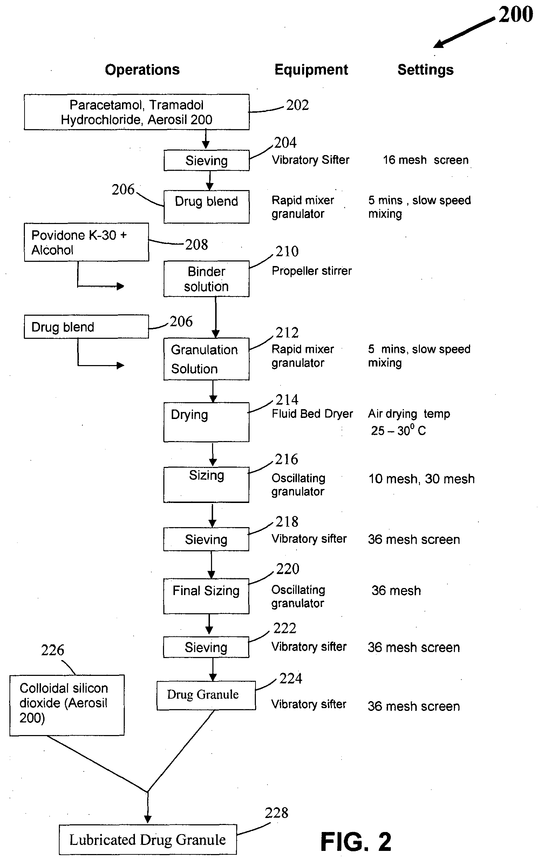

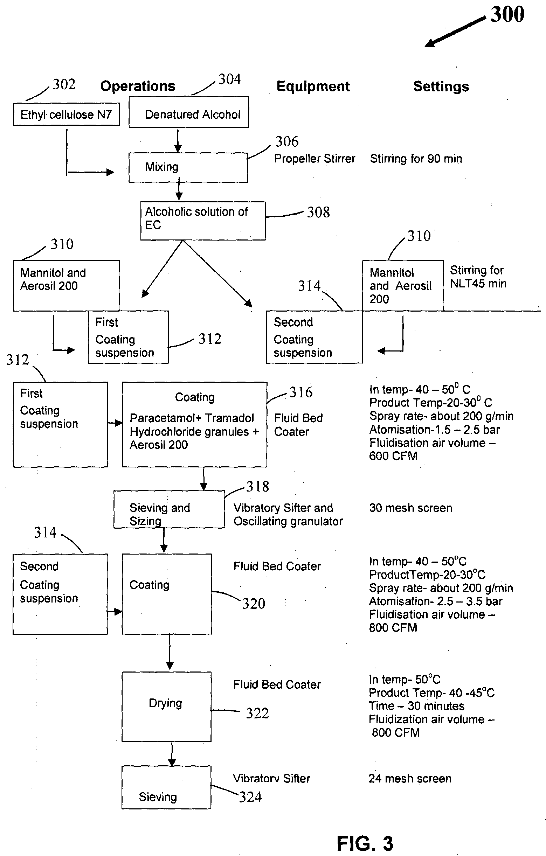

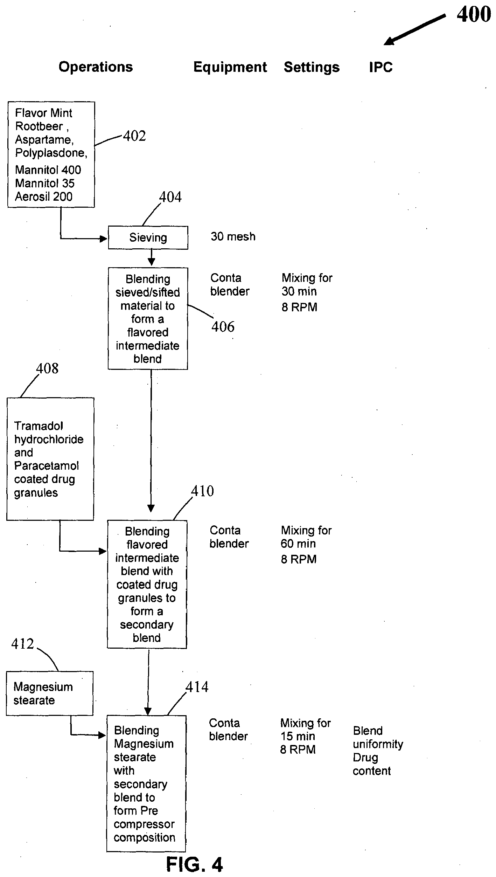

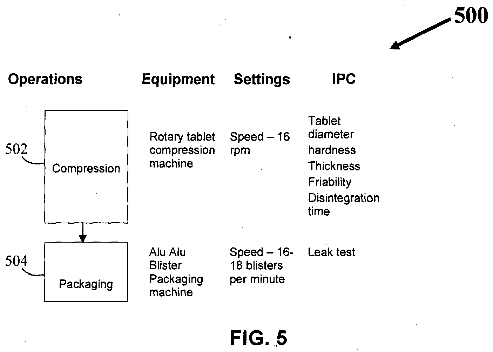

The process 100 is described herein with reference to FIGS. 1, 2, 3, 4 and 5. In FIG. 1, the primary process steps are illustrated, while in FIGS. 2, 3, 4 and 5 are the details of steps illustrated in FIG. 1. Specifically, FIG. 2 illustrates details of the granulation of process of FIG. 1; FIG. 3 illustrates details of taste mask coating of FIG. 1; FIG. 4 illustrates details of preparation of pre-compression composition of process of FIG. 1; and FIG. 5 illustrates details of compression and packing steps of process of FIG. 1 in the below description of the process, the reference numerals are used individually and/or collectively from one or more of the FIGS. 1, 2, 3, 4 and 5.

Referring to FIG. 1, at step 102, the process 100 initiates by mixing tramadol hydrochloride, paracetamol and colloidal silicon dioxide to form a drug blend. Specifically, referring to 202, 204 and 206 of FIG. 2, the drug blend is formed by mixing tramadol hydrochloride, paracetamol and colloidal silicon dioxide by sieving/sifting through a vibratory sifter and then mixing in a rapid mixer granulator. In one embodiment, the sieving/sifting is done through a 16 mesh screen fitted to the vibratory sifter; and mixing is done in the rapid mixer granulator for about 5 minutes. Although a mesh screen of particular dimension is mentioned above, it will be evident to a person skilled in the art to use mesh sieves of varying dimensions for obvious variations. Also, although a particular mixing time is mentioned, it will be evident to a person skilled in the art to use mesh sieves of varying dimensions for obvious variations.

Next, as illustrated at 208 and 210 a binder solution (hereinafter referred as "alcoholic solution of povidone K-30") is prepared by adding povidone K-30 to denatured spirit/isopropyl alcohol. In one embodiment, the alcoholic solution of povidone K-30 is prepared by adding povidone K-30 to a 10 litre tank containing denatured spirit/isopropyl alcohol and stirring with propeller stirrer till the povidone K-30 dissolves in the denatured spirit/isopropyl alcohol.

Now referring again to FIG. 1, at step 104, the process 100 comprises mixing the drug blend and the alcoholic solution of povidone K-30 to form a granulation solution. Referring to Fig. 2, as illustrated at 212, in one embodiment, the granulation solution is formed by mixing drug blend and the alcoholic solution of povidone K-30 in rapid mixer granulator and running the mixer at slow speed with chopper off for 5 minutes. Also, although a particular mixing time is mentioned, it will be evident to a person skilled in the art to use mesh sieves of varying dimensions for obvious variations.

Next at step 106, the process 100 comprises drying, sizing and sieving of the granulation solution to form drug granules. At 214, 216, 218, 220, 222 and 224, illustrated are the details of the steps for forming the drug granules. Herein, at box 214 the granulation solution as illustrated at 212 is dried in fluid bed dryer by first fluidizing it in a current of air of about 25°C to 30°C about till the LOD of these granules is less than 2.5 % to form dried granulates. Thereafter, at 216 the dried granulates as illustrated at 214 are milled by passing through oscillating granulator fitted with 10 mesh (2000µm) screen followed by 30 mesh (600µm) screen to form milled granules. Thereafter, at 218 the milled granules as illustrated at 216 are sieved through 36 mesh (500 µm) screen fitted to vibratory sifter. At 220 and 222 the leftover coarse granules after sieving as illustrated at 218 are re-milled using oscillating granulator fitted with 36 mesh screen followed by sieving through 36 mesh (500 µm) screen fitted to vibratory sifter respectively to form the drug granules at 224. Although, mesh sieves of particular dimensions are mentioned above, it will be evident to a person skilled in the art to use mesh sieves of varying dimensions for obvious variations.

Next at step 108, the process 100 comprises blending the drug granules with colloidal silicon dioxide to form lubricated drug granules. In one embodiment, colloidal silicon dioxide at 226 is sifted through a vibratory sifter fitted with 36 mesh (500 µm) screen along with the drug granules at box 224 to form lubricated drug granules at 228. Although a mesh sieve of particular dimension is mentioned above, it will be evident to a person skilled in the art to use mesh sieves of varying dimensions for obvious variations.

Referring to FIG. 1 again, at step 110, the process 100 comprises spraying a coating solution over lubricated drug granules to form coated drug granules. Specifically, a single taste mask coating solution/composition is used for taste masking both the drugs. The coating solution used in the present invention is a taste masking suspension. In FIG. 3, the step 110 is explained in detail. Ethyl cellulose N7 at 302 and denatured alcohol at 304 are mixed at 306 in a stainless steel tank using a pneumatic stirrer or a propeller stirrer to form alcoholic solution of ethyl cellulose at 308. In one embodiment, as illustrated the stirring is done for 90 minutes to prepare a clear to cloudy solution. On complete dissolution, the alcoholic solution of ethyl cellulose N7 is further divided in two equal parts. To each part of alcoholic solution of ethyl cellulose N7, mannitol 25 is added followed colloidal silicon dioxide, and stirred for not less than 45 minutes to make a homogeneous white suspension, namely, a first coating suspension at 312 and a second coating suspension.

Thereafter, at 316 the first coating suspension is sprayed on to the lubricated drug granules on fluid bed coater at the controlled parameters. As used herein, controlled parameters include an in temperature of about 40°C to about 50°C, product temperature of about 20°C to about 30°C, spraying rate of the first coating suspension part of about 200 grams per minute, atomization of 1.5 bar to 2.5 bar and fluidization of air volume of about 600 CFM. Although particular controlled parameters are mentioned above, it will be evident to a person skilled in the art to modify the controlled parameters based on obvious variations.

Next, at 318, the coated granules of 316 are sifted through 30 mesh screen fitted to the vibratory sifter and the coarser granules are milled through 30 mesh screen fitted to oscillating granulator.

At 320, the second coating suspension of 314 is sprayed on to the coated, sieved and sized granules of 318 on fluid bed coater at the controlled parameters. As used herein, controlled parameters include an in temperature of about 40°C to about 50°C, product temperature of about 20°C to about 30°C, spraying rate of the first coating suspension part of about 200 grams per minute, atomization of 1.5 bar to 2.5 bar and fluidization of air volume of about 800 CFM. Although particular controlled parameters are mentioned above, it will be evident to a person skilled in the art to modify the controlled parameters based on obvious variations.

Next, at 322, the coated granules are dried in fluidized air bed drier at the controlled parameters. As used herein, controlled parameter include an in temperature of about 50°C, product temperature of about 40°C to about 45°C, and fluidization of air volume of about 800 CFM for a period of about 30 minutes. At box 318, the coated granules after drying as illustrated in box 316 are sifted through 24 mesh screen fitted to a vibratory sifter to remove coarser particle. The dried coated granules passed through 24 meshes are considered as good granules for further use and are packaged in two polyethylene bags placed within a drum. Further, two silica gel bags are inserted between the two polyethylene bags. Although a mesh sieve of particular dimension is mentioned above, it will be evident to a person skilled in the art to use mesh sieves of varying dimensions for obvious variations.

At 324, the dried taste masked granules are sifted through a 24 mesh (710 µm) screen fitted to a vibratory sifter to remove coarser particles to finally form the coated drug granules (or taste mask coated drug granules).

Referring to FIG. 1 again, at step 112, the process 100 comprises blending the coated drug granules with mannitol 400, mannitol 35, a disintegrant, a sweetener, a flavorant, colloidal silicon dioxide and magnesium stearate i.e., a lubricant to form a pre- compression composition. As illustrated in FIG. 4, mannitol 400, the mannitol 35, the sweetener (aspartame), the flavorant (flavor mint rootbeer), the disintegrant (polyplasdone) and colloidal silicon dioxide at 402, are sifted/sieved through a 30 mesh screen at 404. Thereafter, at 406, the sieved material from 404 blended in a conta blender for about 30 minutes at about 8 revolutions per minute to form a flavoured intermediate blend.

Further, at 410, the flavoured intermediate blend lend is blended with the coated drug granules received after step 110 in conta- blender for 60 minute at 8 revolution per minute (RPM) to form a secondary blend.

Thereafter, magnesium stearate (lubricant) at 414 is sifted through 36 mesh screen and introduced into the secondary blend to form a lubricated pre-compression composition at 414. In one embodiment, the lubrication at 414 is performed in a conta blender for about 15 minutes at 8 revolutions per minute.

Referring to FIG. 1 again, at step 114, the process 100 comprises compressing the pre-compression composition to form the tramadol hydrochloride and paracetamol orally disintegrating composition of the present invention. Referring to Fig. 5, at 502, the compression is performed using rotary tablet compression machine at a speed of about 16 revolutions per minute. Also, referring to Table 1 below illustrated below are the parameters by which the lubricated pre- compression composition is compressed:-

**TABLE 1**

| **Sr. No.** | **Parameters** | **Standard** | **Limits** |
|---|---|---|---|
| 1 | Appearance | White to off white round flat tablet with concave depression in the center on both the surface having mint odour. | |
| 2 | Weight of 20 tablets | 24.00 g | 22.80 - 25.20 g |
| 3 | Individual tablet weight | 1200 mg | 1140 - 1260 mg |
| 4 | Thickness | 5.0 mm | 4.70 - 5.3 mm |
| 5 | Hardness | 50 N | NLT 40 N |
| 6 | Disintegration time | NMT 3 min at 37°C | NMT 3 min at 37°C |
| 7 | Friability | NMT 1.0 % | NMT 1.0 % |

Although particular compression parameters are mentioned above, it will be evident to a person skilled in the art to modify parameters based on obvious variations.

The process may further comprise packing the compositions. Referring to FIG. 5, the details of the packing are mentioned. In an embodiment, the packing is done using ALU-ALU blister material at a production speed of about 16 to about 18 blisters per minute.

The description of the tramadol hydrochloride and paracetamol orally disintegrating composition of the present invention is further illustrated by the following non-limiting example. However, a person skilled in the art would recognize that, the specific example is intended to illustrate, not limit, the scope of the present invention.

### EXAMPLE 1

In Example, a process for preparing a tramadol hydrochloride 37.5 mg and paracetamol 325 mg orally disintegrating tablets is described. The process for preparing tramadol hydrochloride and paracetamol composition was initiated by mixing 6 kilogram (Kg) of tramadol hydrochloride, 52 Kg of paracetamol and 0.28 Kg of colloidal silicon dioxide by sieving/sifting through a 16 mesh screen fitted to the vibratory sifter and followed by mixing in the rapid mixer granulator for about 5 minutes form a drug blend. Thereafter the drug blend is mixed with alcoholic solution of povidone K-30 in rapid mixer granulator and running the mixer at slow speed with chopper off for 5 minutes to form a granulation solution. The alcoholic solution of povidone K-30 was formed by adding 0.56 Kg of povidone K-30 with 4 Kg of denatured spirit and stirring with propeller stirrer till the povidone K-30 dissolves in the denatured spirit.

Next, the granulation solution is dried in fluid bed dryer by first fluidizing it in a current of air of about 25°C to 30°C about till the LOD of these granules is less than 2.5 % to form dried granulates. Thereafter, the dried granulates are milled by passing through oscillating granulator fitted with 10 mesh (2000µm) screen followed by 30 mesh (600µm) screen to form milled granules. Thereafter, the milled granules are sieved through 36 mesh (500 µm) screen fitted to vibratory sifter. Next, the leftover coarse granules after sieving are re-milled using oscillating granulator fitted with 36 mesh screen followed by sieving through 36 mesh (500 µm) screen fitted to vibratory sifter respectively to form the drug granules. Next, the drug granules is mixed with 0.40 Kg of colloidal silicon dioxide by sifting through a vibratory sifter fitted with 36 mesh (500 µm) screen to form lubricated drug granules.

Next, taste mask coating is performed with the first coating suspension and the second coating suspension to form coated drug granules. The coating suspension was formed by mixing 9.83 Kg of ethyl cellulose N7 with 181.55 Kg of denatured spirit in a stainless steel tank using pneumatic stirrer for 90 minutes. Thereafter on complete dissolution of ethyl cellulose, the solution is further divided in two equal parts namely first coating suspension and the second coating suspension. To each parts of suspension 1.67 kg of mannitol 25 is added followed by 0.67 kg of colloidal silicon dioxide and stirred for not less than 45 minutes to make homogeneous white suspension. The first coating suspension is sprayed on to the lubricated drug granules on fluid bed coater at the controlled parameters to form coated granules. As used herein, controlled parameters include an in temperature of about 40°C to about 50°C, product temperature of about 20°C to about 30°C, spraying rate of the first coating suspension part of about 200 grams per minute, atomization of 1.5 bar to 2.5 bar and fluidization of air volume of about 600 CFM. Next, the coated granules are sifted through 30 mesh screen fitted to the vibratory sifter and the coarser granules are milled through 30 mesh screen fitted to oscillating granulator. Thereafter, the second coating suspension is sprayed on to the coated granules on fluid bed coater at the controlled parameters. As used herein, controlled parameters include an in temperature of about 40°C to about 50°C, product temperature of about 20°C to about 30°C, spraying rate of the first coating suspension part of about 200 grams per minute, atomization of 1.5 bar to 2.5 bar and fluidization of air volume of about 800 CFM.

Next, the coated granules are dried in fluidized air bed drier at the controlled parameters. As used herein, controlled parameter include an in temperature of about 50°C, product temperature of about 40°C to about 45°C, and fluidization of air volume of about 800 CFM for a period of about 30 minutes. Thereafter, the coated granules after drying are sifted through 24 mesh screen fitted to a vibratory sifter to remove coarser particle. The dried coated granules passed through 24 meshes are considered as good granules for further use and are packaged in two polyethylene bags placed within a drum. Further, two silica gel bags are inserted between the two polyethylene bags.

Thereafter, the dried taste masked granules are sifted through a 24 mesh (710 µm) screen fitted to a vibratory sifter to remove coarser particles to finally form the coated drug granules (or taste mask coated drug granules). Next, 33.75 Kg of mannitol 400, 33.75 Kg of mannitol 35, 15 Kg of crosspovidone (a disintegrant), 6 Kg of aspartame (a sweetener), 1.62 Kg of flavor-root beer mint (a flavorant) and 0.75 Kg of colloidal silicon dioxide are sifted/sieved through a 30 mesh screen and blended in a conta blender for about 30 minutes at about 8 revolutions per minute to form a flavoured intermediate blend. Next, the flavoured intermediate blend is blended with 57.63 Kg of coated drug granules in conta- blender for 60 minute at 8 revolution per minute (RPM) to form a secondary blend.

Thereafter, 1.50 Kg of magnesium stearate (a lubricant) is sifted through 36 mesh screen and introduced into the secondary blend to form a lubricated pre- compression composition. Herein, the lubrication is performed in a conta blender for about 15 minutes at 8 revolutions per minute.

Finally, the pre- compression composition was compressed to form the tramadol hydrochloride and paracetamol orally disintegrating composition. The solvent, i.e., denatured spirit does not remain in the final composition. In Table 2 below, the different ingredients of the tramadol hydrochloride and paracetamol orally disintegrating composition are depicted along with details on pharmacopeial reference, the vendor from whom the ingredient was obtained, percent by weight of each ingredient and weight in mg of each ingredient.

**TABLE 2**

| **Component** | **Pharmacopoeia** | **Vendor** | **% Formula** | **Quantity mg/ tab** |
|---|---|---|---|---|
| **Taste Mask granules** | | | | |
| **Granulation** | | | | |
| Paracetamol | Ph.Eur | Sri Krishna Pharmaceuticals | 27.08 | 325.0 |
| Tramadol Hydrochloride | Ph.Eur | Jubilant Organosys | 3.13 | 37.50 |
| Aerosil 200 | EP / BP | Evonik | 0.15 | 1.75 |
| PVP K-30 | EP / BP | BASF | 0.29 | 3.50 |

| **Lubrication of Granules** | | | | |
|---|---|---|---|---|
| Aerosil 200 | EP / BP | Evonik | 0.21 | 2.50 |

| **Coating** | | | | |
|---|---|---|---|---|
| Ethyl cellulose N7 | EP / BP | Colorcon / Aqualon | 5.12 | 61.44 |
| Mannitol fine (Pearlitol 25) | EP / BP | Roquette | 1.74 | 20.88 |
| Aerosil 200 | EP / BP | Roquette | 0.7 | 8.38 |
| Denatured spirit* / Isopropyl alcohol | In house | Oasis Chemicals India | Q.S | Q.S |

| **Orally disintegrating Tablet composition** | | | | |
|---|---|---|---|---|
| Taste Mask granules | In house | Athena DDS | | 461.00 |
| Mannitol granular (Pearlitol 400) | EP / BP | Roquette | 22.5 | 270.00 |
| Mannitol powder (Pearlitol 35) | EP / BP | Roquette | 22.5 | 270.00 |
| Polyplasdone XL | EP / BP | BASF | 10 | 120.00 |
| Aspartame | EP / BP | Nutrasweets | 4 | 48.00 |
| Flavor- Mint Rootbeer | EP / BP | Pharmarome | 1.08 | 13.00 |
| Aerosil 200 | EP / BP | Evonik | 0.5 | 6.00 |
| Magnesium Stearate | EP / BP | Ferro, Portugal | 1.0 | 12.00 |
| Total | | | 100 | 1200 |

Specifically, the process and composition details described in example and Table 2 is used for preparing tramadol hydrochloride 37.5 mg and paracetamol 325 mg orally disintegrating tablets (hereinafter referred to as tramadol hydrochloride 37.5 mg and paracetamol 325 mg ODT). Multiple experiments were conducted to form tramadol hydrochloride 37.5 mg and paracetamol 325 mg , i.e, to form tramadol hydrochloride 37.5 mg and paracetamol 325 mg ODT comprising 37.5 mg of tramadol hydrochloride and 325 mg of paracetamol . For example, experiment for batches 1, 2 and 3 were conducted for forming the final tramadol hydrochloride 37.5 mg and paracetamol 325 mg ODT. All such properties for the different batches for tramadol hydrochloride content and paracetamol content are illustrated in Tables 3 and 4 below respectively.

**TABLE 3**

| **Tramadol Hydrochloride content** | | | |
|---|---|---|---|
| **Batch No.** | 1 | 2 | 3 |
| **Average weight 1200 mg ± 5% (1140 - 1260)** | 1200.5 mg | 1208.7 mg | 1210.2 mg |
| **Assay (Limit 90 - 110%)** | 37.58 mg / Tablet (100.18%) | 38.47 mg/tablet (102.59%) | 37.35 mg (99.59%) |
| **Uniformity of content (85 - 115%, AV -15)** | AV = 6.67 | AV = 8.56 | AV = 3.64 |
| | Mean= 100.39 % | Mean =103.84% | Mean =100.79 % |
| | Min = 96.13 % | Min = 101.57 % | Min = 98.91 % |
| | Max = 105.20 % | Max = 109.04 % | Max = 103.84 % |
| | % RSD = 2.77 | % RSD = 2.50 | % RSD = 1.51 |
| **Dissolution (Paddle, 50 rpm, 500 ml, 0.1N HCl) Limit NLT 80% in 30 minutes** | 100.91 % | 99.26 % | 100.20 % |
| | (N = 6 vessels) | (N = 6 vessels) | (N = 6 vessels) |
| | Min = 99.60 % | Min = 96.51% | Min = 98.85 % |
| | Max = 102.35 % | Max = 102.51 % | Max = 101.47 % |
| | % RSD = 0.87 | % RSD = 2.26 | % RSD = 0.93 |
| **Related substance** | | | |
| **Tramadol Impurity A - NMT 0.3%** | NIL | NIL | NIL |
| **Unspecified Impurity - NMT 0.2%** | 0.1% | 0.1% | 0.1% |
| **Total Impurity - NMT 1.0%** | 0.2% | 0.2% | 0.2% |

**TABLE 4**

| **Paracetamol content** | | | |
|---|---|---|---|
| **Batch No.** | 1 | 2 | 3 |
| **Assay (Limit 90** - **110**%) | 321.9 mg / Tablet (99.05%) | 324.68 mg/tablet (99.9%) | 321.1 mg (98.8%) |
| **Dissolution (Paddle, 50 rpm, 500 ml, 0.1N HCl) Limit NLT 80% in 30 minutes** | 95.66 % | 95.08 % | 94.90 % |
| | (N = 6 vessels) | (N = 6 vessels) | (N = 6 vessels) |
| | Min = 94.23 % | Min = 91.70 % | Min = 93.61 % |
| | Max = 96.77 % | Max = 102.05 % | Max = 95.78 % |
| | % RSD = 0.92 | % RSD = 3.89 | % RSD = 0.86 |
| **Related substance** | | | |
| **4 - Aminophenol Impurity - NMT 0.1%** | NIL | NIL | NIL |
| **Unspecified Impurity - NMT 0**.**25**% | 0.01% | 0.01% | 0.01% |
| **Total Impurity - NMT 0.5%** | 0.01% | 0.01% | 0.01% |

## Claims

1. A tramadol hydrochloride and paracetamol orally disintegrating composition, comprising:
about 2.5 to about 5 percent by weight of a tramadol hydrochloride;
about 25 to about 30 percent by weight of paracetamol;
about 1.25 to about 1.75 percent by weight of colloidal silicon dioxide;
about 0.25 to about 0.35 percent by weight of povidone K-30;
about 4.5 to about 5.5 percent by weight of ethyl cellulose N7;
about 1.5 to about 2 percent by weight of mannitol 25;
about 20 to about 25 percent by weight of mannitol 400;
about 20 to about 25 percent by weight of mannitol 35;
about 9 to about 11 percent by weight of a disintegrant;
about 3 to about 5 percent by weight of a sweetener;
about 1 to about 1.5 percent by weight of a flavorant; and
about 0.5 to about 1.5 percent by weight of a lubricant.

2. The tramadol hydrochloride and paracetamol orally disintegrating composition of claim 1, wherein the lubricant is magnesium stearate.

3. The tramadol hydrochloride and paracetamol orally disintegrating composition of claim 1, wherein the disintegrant is polyplasdone XL.

4. The tramadol hydrochloride and paracetamol orally disintegrating composition of claim 1, wherein the sweetener is aspartame.

5. The tramadol hydrochloride and paracetamol orally disintegrating composition of claim 1, wherein the flavorant is flavor-mint rootbeer.

6. The tramadol hydrochloride and paracetamol orally disintegrating composition of claim 1, wherein the tramadol hydrochloride and paracetamol orally disintegrating composition is in form of an orally disintegrating tablet having a disintegrating time of about 3 minutes and a hardness of about 40 Newton to 50 Newton.

7. The tramadol hydrochloride and paracetamol orally disintegrating composition of claim 1, wherein a single coating solution of ethyl cellulose N7 is used for coating both tramadol hydrochloride and paracetamol.

8. A process for preparation of a tramadol hydrochloride and paracetamol orally disintegrating composition, comprising:
mixing tramadol hydrochloride, paracetamol and colloidal silicon dioxide to form a drug blend;
mixing the drug blend and alcoholic solution of povidone K-30 to form a granulation solution;
drying, sizing and sieving the granulation solution to form drug granules;
blending the drug granules with colloidal silicon dioxide to form lubricated drug granules;
spraying a taste masking coating solution comprising ethyl cellulose N7 over lubricated drug granules to form coated drug granules;
blending the coated drug granules with mannitol 25, mannitol 400, mannitol 35, a disintegrant, a sweetener, a flavorant, colloidal silicon dioxide and a lubricant to form a pre-compression composition; and
compressing the pre- compression composition.

9. The process of claim 8, further comprising packing the tramadol hydrochloride and paracetamol orally disintegrating composition.

10. The process of claim 8, wherein the tramadol hydrochloride and paracetamol orally disintegrating composition comprises
about 2.5 to about 5 percent by weight of tramadol hydrochloride;
about 25 to about 30 percent by weight of paracetamol;
about 1.25 to about 1.75 percent by weight of colloidal silicon dioxide;
about 0.25 to about 0.35 percent by weight of povidone K-30;
about 4.5 to about 5.5 percent by weight of ethyl cellulose N7;
about 1.5 to about 2 percent by weight of mannitol 25;
about 20 to about 25 percent by weight of mannitol 400;
about 20 to about 25 percent by weight of mannitol 35;
about 9 to about 11 percent by weight of a disintegrant;
about 3 to about 5 percent by weight of a sweetener;
about 1 to about 1.5 percent by weight of a flavorant; and
about 0.5 to about 1.5 percent by weight of a lubricant.

11. The process of claim 10, wherein the lubricant is magnesium stearate.

12. The process of claim 10, wherein the disintegrant is polyplasdone XL.

13. The process of claim 10, wherein the sweetener is aspartame.

14. The process of claim 10, wherein the flavorant is flavor-mint rootbeer.

15. The process of claim 10, wherein step of forming coated drug granules is performed in a fluid bed coater.

16. The process of claim 10, wherein a single coating solution is used for coating both tramadol hydrochloride and paracetamol.

## Patentansprüche

1. Zusammensetzung zur oralen Desintegration von Tramadolhydrochlorid und Paracetamol, umfassend:
etwa 2,5 bis etwa 5 Gewichtsprozent Tramadolhydrochlorid;
etwa 25 bis etwa 30 Gewichtsprozent Paracetamol;
etwa 1,25 bis etwa 1,75 Gewichtsprozent kolloidales Siliciumdioxid;
etwa 0,25 bis etwa 0,35 Gewichtsprozent Povidon K-30;
etwa 4,5 bis etwa 5,5 Gewichtsprozent Ethylcellulose N7;
etwa 1,5 bis etwa 2 Gewichtsprozent Mannitol 25;
etwa 20 bis etwa 25 Gewichtsprozent Mannitol 400;
etwa 20 bis etwa 25 Gewichtsprozent Mannitol 35;
etwa 9 bis etwa 11 Gewichtsprozent eines Sprengmittels;
etwa 3 bis etwa 5 Gewichtsprozent eines Süßstoffes;
etwa 1 bis etwa 1,5 Gewichtsprozent eines Aromastoffes; und
etwa 0,5 bis etwa 1,5 Gewichtsprozent eines Schmiermittels.

2. Zusammensetzung zur oralen Desintegration von Tramadolhydrochlorid und Paracetamol nach Anspruch 1, wobei das Schmiermittel Magnesiumstearat ist.

3. Zusammensetzung zur oralen Desintegration von Tramadolhydrochlorid und Paracetamol nach Anspruch 1, wobei das Sprengmittel Polyplasdon XL ist.

4. Zusammensetzung zur oralen Desintegration von Tramadolhydrochlorid und Paracetamol nach Anspruch 1, wobei der Süßstoff Aspartam ist.

5. Zusammensetzung zur oralen Desintegration von Tramadolhydrochlorid und Paracetamol nach Anspruch 1, wobei der Aromastoff Rootbeer mit Minzgeschmack ist.

6. Zusammensetzung zur oralen Desintegration von Tramadolhydrochlorid und Paracetamol nach Anspruch 1, wobei die Zusammensetzung zur oralen Desintegration von Tramadolhydrochlorid und Paracetamol in Form einer oral desintegrierenden Tablette mit einer Desintegrationszeit von etwa 3 min und einer Härte von etwa 40 Newton bis 50 Newton vorliegt.

7. Zusammensetzung zur oralen Desintegration von Tramadolhydrochlorid und Paracetamol nach Anspruch 1, wobei eine einzige Beschichtungslösung von Ethylcellulose N7 zur Beschichtung von sowohl Tramadolhydrochlorid als auch Paracetamol verwendet wird.

8. Verfahren zur Herstellung einer Zusammensetzung zur oralen Desintegration von Tramadolhydrochlorid und Paracetamol, umfassend:
Vermischen von Tramadolhydrochlorid, Paracetamol und kolloidalem Siliciumdioxid zur Bildung eines Arzneimittelgemisches;
Vermischen des Arzneimittelgemisches und einer alkoholischen Lösung von Povidon K-30 zur Bildung einer Granulierungslösung;
Trocknen, Ausmessen und Sieben der Granulierungslösung zur Bildung von Arzneimittelgranula;
Vermischen der Arzneimittelgranula mit kolloidalem Siliciumdioxid zur Bildung von geschmierten Arzneimittelgranula;
Sprühen einer geschmacksüberdeckenden Beschichtungslösung umfassend Ethylcellulose N7 über geschmierte Arzneimittelgranula zur Bildung von beschichteten Arzneimittelgranula;
Vermischen der beschichteten Arzneimittelgranula mit Mannitol 25, Mannitol 400, Mannitol 35, einem Sprengmittel, einem Süßstoff, einem Aromastoff, kolloidalem Siliciumdioxid und einem Schmiermittel zur Bildung einer vorgepressten Zusammensetzung; und
Pressen der vorgepressten Zusammensetzung.

9. Verfahren nach Anspruch 8, das weiter das Verpacken der Zusammensetzung zur oralen Desintegration von Tramadolhydrochlorid und Paracetamol umfasst.

10. Verfahren nach Anspruch 8, wobei die Zusammensetzung zur oralen Desintegration von Tramadolhydrochlorid und Paracetamol Folgendes umfasst:
etwa 2,5 bis etwa 5 Gewichtsprozent Tramadolhydrochlorid;
etwa 25 bis etwa 30 Gewichtsprozent Paracetamol;
etwa 1,25 bis etwa 1,75 Gewichtsprozent kolloidales Siliciumdioxid;
etwa 0,25 bis etwa 0,35 Gewichtsprozent Povidon K-30;
etwa 4,5 bis etwa 5,5 Gewichtsprozent Ethylcellulose N7;
etwa 1,5 bis etwa 2 Gewichtsprozent Mannitol 25;
etwa 20 bis etwa 25 Gewichtsprozent Mannitol 400;
etwa 20 bis etwa 25 Gewichtsprozent Mannitol 35;
etwa 9 bis etwa 11 Gewichtsprozent eines Sprengmittels;
etwa 3 bis etwa 5 Gewichtsprozent eines Süßstoffes;
etwa 1 bis etwa 1,5 Gewichtsprozent eines Aromastoffes; und
etwa 0,5 bis etwa 1,5 Gewichtsprozent eines Schmiermittels.

11. Verfahren nach Anspruch 10, wobei das Schmiermittel Magnesiumstearat ist.

12. Verfahren nach Anspruch 10, wobei das Sprengmittel Polyplasdon XL ist.

13. Verfahren nach Anspruch 10, wobei der Süßstoff Aspartam ist.

14. Verfahren nach Anspruch 10, wobei der Aromastoff Rootbeer mit Minzgeschmack ist.

15. Verfahren nach Anspruch 10, wobei der Schritt der Bildung von beschichteten Arzneimittelgranula in einem Wirbelbettbeschichter durchgeführt wird.

16. Verfahren nach Anspruch 10, wobei eine einzige Beschichtungslösung zur Beschichtung von sowohl Tramadolhydrochlorid als auch Paracetamol verwendet wird.

## Revendications

1. Composition à désintégration orale à base de chlorhydrate de tramadol et de paracétamol renfermant :
environ 2,5 pourcent à environ 5 pourcent en poids de chlorhydrate de tramadol,
environ 25 pourcent à environ 30 pourcent en poids de paracétamol,
environ 1,25 pourcent à environ 1,75 pourcent en poids de dioxyde de silicium colloïdal,
environ 0,25 pourcent à environ 0,35 pourcent en poids de povidone K-30,
environ 4,5 pourcent à environ 5,5 pourcent en poids d'éthyl cellulose N7,
environ 1,5 pourcent à environ 2 pourcent en poids de mannitol 25,
environ 20 pourcent à environ 25 pourcent en poids de mannitol 400,
environ 20 pourcent à environ 25 pourcent en poids de mannitol 35,
environ 9 pourcent à environ 11 pourcent en poids d'un agent de désintégration,
environ 3 pourcent à environ 5 pourcent en poids d'un agent édulcorant,
environ 1 pourcent à environ 1,5 pourcent en poids d'un arôme, et
environ 0,5 pourcent à environ 1,5 pourcent en poids d'un lubrifiant.

2. Composition à désintégration orale à base de chlorhydrate de tramadol et de paracétamol dans laquelle le lubrifiant est le stéarate de magnésium.

3. Composition à désintégration orale à base de chlorhydrate de tramadol et de paracétamol dans laquelle l'agent de désintégration est le polyplasdone XL.

4. Composition à désintégration orale à base de chlorhydrate de tramadol et de paracétamol dans laquelle l'agent édulcorant est l'aspartame.

5. Composition à désintégration orale à base de chlorhydrate de tramadol et de paracétamol dans laquelle l'arôme est un arôme à base de menthe-racinette.

6. Composition à désintégration orale à base de chlorhydrate de tramadol et de paracétamol conforme à la revendication 1, se présentant sous la forme d'un comprimé à désintégration orale ayant un temps de désintégration d'environ 3 minutes et une dureté d'environ 40 Newton à environ 50 Newton.

7. Composition à désintégration orale à base de chlorhydrate de tramadol et de paracétamol conforme à la revendication 1, dans laquelle une solution de revêtement unique d'éthyl cellulose N7 est utilisée pour revêtir le chlorhydrate de tramadol et le paracétamol.

8. Procédé d'obtention d'une composition à désintégration orale à base de chlorhydrate de tramadol et de paracétamol comprenant des étapes consistant à :
mélanger du chlorhydrate de tramadol, du paracétamol et du dioxyde de silicium colloïdal pour former un mélange médicamenteux,
mélanger le mélange médicamenteux et une solution alcoolique de povidone K-30 pour former une solution de granulation,
sécher, cribler et tamiser la solution de granulation pour former des granulés médicamenteux,
mélanger des granulés médicamenteux avec du dioxyde de silicium colloïdal pour former des granulés médicamenteux lubrifiés,
pulvériser une solution de revêtement masquant le goût renfermant de l'éthyl cellulose N7 sur les granulés médicamenteux lubrifiés pour former des granulés médicamenteux revêtus,
mélanger les granulés médicamenteux revêtus avec du mannitol 25, du mannitol 400, du mannitol 35, un agent de désintégration, un agent édulcorant, un arôme, du dioxyde de silicium colloïdal et un lubrifiant pour former une composition de précompression, et
comprimer la composition de précompression.

9. Procédé conforme à la revendication 8,
comprenant en outre une étape consistant à emballer la composition à désintégration orale à base de chlorhydrate de tramadol et de paracétamol.

10. Procédé conforme à la revendication 8,
selon lequel la composition à désintégration orale à base de chorydrate de tramadol et de paracétamol renferme :
environ 2,5 pourcent à environ 5 pourcent en poids de chlorydrate de tramadol,
environ 25 pourcent environ 30 pourcent en poids de paracétamol,
environ 1,25 pourcent à environ 1,75 pourcent en poids de dioxyde de silicium colloïdal,
environ 0,25 pourcent à environ 0,35 pourcent en poids de povidone K-30,
environ 4,5 pourcent à environ 5,5 pourcent en poids d'éthyl cellulose N7,
environ 1,5 pourcent à environ 2 pourcent en poids de mannitol 25,
environ 20 pourcent à environ 25 pourcent en poids de mannitol 400,
environ 20 pourcent à environ 25 pourcent en poids de mannitol 35,
environ 9 pourcent à environ 11 pourcent en poids d'un agent de désintégration,
environ 3 pourcent à environ 5 pourcent en poids d'un agent édulcorant,
environ 1 pourcent à environ 1,5 pourcent en poids d'un arôme, et
environ 0,5 pourcent à environ 1,5 pourcent en poids d'un lubrifiant.

11. Procédé conforme à la revendication 10, selon lequel le lubrifiant est le stérate de magnésium.

12. Procédé conforme à la revendication 10, selon lequel l'agent de désintégration est le polyplasdone XL.

13. Procédé conforme à la revendication 10, selon lequel l'agent édulcorant est l'aspartame.

14. Procédé conforme à la revendication 10, selon lequel l'arôme est un arôme à base de menthe-racinette.

15. Procédé conforme à la revendication 10, selon lequel l'étape de formation de granulés médicamenteux revêtus est mise en oeuvre dans un dispositif de revêtement à lit fluidisé.

16. Procédé conforme à la revendication 10, selon lequel une solution de revêtement unique est utilisée pour revêtir le chlorhydrate de tramadol et le paracétamol.
